# EUROPEAN PATENT SPECIFICATION

(11) **EP 2 092 336 B1**
(45) Date of publication and mention of the grant of the patent: **29.08.2012**
(21) Application number: 07847296.6
(22) Date of filing: 23.11.2007
(51) Int. Cl.: G01N 33/543

(54) **CAPTURE OF MYCOBACTERIA LIKE MICRO-ORGANISMS**
EINFANG MYKOBAKTERIEN-ÄHNLICHER MIKROORGANISMEN
CAPTURE DE MICROORGANISMES DE TYPE MYCOBACTÉRIES

(30) Priority: 29.11.2006 GB 0623866; 07.06.2007 GB 0710977
(43) Date of publication of application: 26.08.2009
(73) Proprietor: Microsens Medtech Ltd, London NW1 0NH (GB)
(72) Inventor: WILSON, Stuart, Mark, London NW1 0NH (GB); STANLEY, Christopher John, London NW1 0NH (GB)
(74) Representative: Smart, Peter John
(86) International application number: PCT/EP2007/062732
(87) International publication number: WO 2008/065047

(56) References cited:
- WO-A-99/36781
- WO-A-2006/034385
- STRATMANN J ET AL: "Development of a peptide-mediated capture PCR for detection of Mycobacterium avium subsp. paratuberculosis in milk" JOURNAL OF CLINICAL MICROBIOLOGY, WASHINGTON, DC, US, vol. 40, no. 11, November 2002 (2002-11), pages 4244-4250, XP002265157 ISSN: 0095-1137 cited in the application

## Description

The present invention relates to the capture to a surface hydrophobic micro-organism, being mycobacteria, and to subsequent processing such as assays for their presence or identification.

Pathogenic mycobacteria are responsible for several severe infectious diseases in humans and animals. The mycobacteria are characterised by a hydrophobic, waxy coat comprising mycolic acid or related compounds. Mycolic acids are complex hydroxylated branched chain fatty acids, typically having hydrocarbon chains with a chain length in the range C₇₇₋₁₈, which causes severe problems in sample handling, causing the bacteria to clump forming cords and to float on the surface of liquids and to be resistant to centrifugation. The hydrocarbon chains may or may not contain sparse oxygenated groups such as hydroxyl, methoxy, keto or carboxyl. Pathogenic mycobacteria include *Mycobacterium tuberculosis,* which is the causative agent of TB, the mycobacteria of the MAC complex (primarily *M. avium* and *M. intracellulare*) which are opportunistic pathogens in AIDS patients, *M. paratuberculosis,* which causes bowel inflammation, *M. leprae* causing leprosy, *M. kansasii, M marinum, M. fortuitum* complex, and many others. There are also many other non-pathogenic mycobacteria, including *M. smegmatis.* Also, other members of the *Mycolata* family have similar hydrophobic coat components. In some, the chain length of the hydrophobic fatty acids is shorter than in the mycobacteria, at around 50 carbon atoms, and in others around 30.

In order to diagnose mycobacterial infections such as tuberculosis, the presence of the organism must be demonstrated by microscopy, culture or molecular methods such as PCR. Although microscopy can be done directly from the biological sample, it is more usual to first isolate and concentrate the mycobacteria from the biological specimens prior to analysis. Biological samples can include sputum, urine, blood, bronchial lavage etc. One of the most common specimen types delivered for diagnosis is sputum. Sputum presents unique problems for bacteriology. Sputum is heterogenous in nature and can be bloody, purulent, and viscous. It can also be contaminated with other micro-organisms eg. *Pseudomonas*.

Commonly, sputum is thinned and at the same time decontaminated by the use of various pre-treatments. These treatments include the use of 0.25-0.5 M sodium hydroxide with or without N-acetyl L-cysteine, sodium dodecyl sulphate, oxalic acid or trisodium phosphate. Treatment times can be 20-120 minutes. These treatments are designed to thin the sputum and kill the majority of contaminating organisms. Mycobacteria have a thick waxy coat and are more resistant to such treatments. Even so, it is estimated that up to 60% of *Mycobacterium tuberculosis* are killed or rendered non-viable by this treatment. In addition, because the *Mycobacterium tuberculosis* and other members of the family grow so slowly, the growth of contaminating organisms that are not killed by this treatment is still a problem with a high percentage of cultures being overgrown by the fast-growing contaminants.

After treatment with the harsh decontaminants the sample is centrifuged to concentrate the mycobacteria which are then analysed by microscopy, culture or molecular amplification. This centrifugation step introduces a risk of infection to the laboratory staff as the contents of any tube that cracks or breaks during the centrifugation may be aerosolised and contaminate the environment. The centrifugation also introduces a bottle-neck in the sample processing as only a limited number of samples can be centrifuged at any one time. In addition, the centrifugation pellets all material that was rendered denatured and insoluble by the harsh decontamination procedure and very large pellets can be obtained which pose problems for microscopy or molecular methods.

Because of the problems listed above with the current decontamination and concentration approaches it would be extremely useful if the mycobacteria could be captured directly from the biological sample. It would be helpful if this procedure removed some or all of the contaminating organisms such that the chemical decontamination is not needed or could be performed with less harsh conditions. This would also enhance the survival of the purified mycobacteria and increase the sensitivity of subsequent tests.

In other applications distinct from sample processing it might also be useful to bind the mycobacteria to a solid surface to allow easy concentration or manipulation of the organisms e.g. capture and washing of the mycobacteria from a phage solution to remove exogenous non-infecting phage or capture and transfer of the mycobacteria from one solution to another.

Methods of capturing mycobacteria to solid surfaces have previously been proposed, including the use of bound phage or phage derived binding peptides immobilised on beads and acting as capture agents (Stratmann et al; J Clin Microbiol. 2002 November; 40(11): 4244-4250) and including isolation of *M. paratuberculosis* from milk by the use of antibody coated beads (Grant I. R. et al; Appl Environ Microbiol. 1998 Sep; 64(9):3153-8). However, such a method may be too expensive for extensive use, especially in less developed countries, and may be over specific in that not all desired bacteria will be captured and involves protein-based molecules that are susceptible to proteases, denaturation and harsh chemicals.

According to Hetland G. et al., Immunology 1994, 82, 445-449, it is possible to coat latex microbeads with BCG by incubation of the beads with cultured and separated bacteria. However, this is unlikely to be effective to capture efficiently such bacteria from a biological sample containing other hydrophobic organisms or materials.

WO 2006/034385 discloses a polydiallyl-dimethylammonium (PDDA) coated slide for immobilising Sitta cells

WO 99/36781 discloses a chromatography device for use with whole blood samples such as to aggregate red blood cells and to pass plasma or serum.

We have observed that poly diallyldimethyl ammonium chloride (p-DADMAC) binds mycobacteria to carboxylic acid micro-beads. Without being bound by the following theory, we believe that the backbone chain of the p-DADMAC hydrophobically interacts with the waxy coat of the mycobateria and the positive charge in the backbone of p-DADMAC can also interact with negative charges on the surface of the mycobacteria, the p-DADMAC then interacts ionically through its pendant quaternary ammonium groups with the carboxylic acids of the micro-beads. We have also observed that p-DADMAC coated surfaces such as plastics and glass can bind mycobacteria directly.

Thus mycobacteria can be either captured directly to p-PADMAC coated surfaces or can be captured to a surface directly.

The present invention now therefore provides in a first aspect a method for the capture from a sample of micro-organisms having a hydrophobic surface, said micro-organisms being Mycobacteria, which method comprises contacting the micro-organisms with a capture reagent, which capture reagent has both a hydrophobic character whereby the capture reagent binds said Mycobacteria by hydrophobic interaction therewith and a polar character and which is poly-diallyldimethyl ammonium chloride (pDADMAC), said capture reagent either being present on a surface and capturing said Mycobacteria thereto, or being present in solution, said method then further comprising capturing said Mycobacteria to a surface by binding said capture reagent to said surface by polar interaction between said surface and said capture reagent.

Preferably, the above method is conducted using the capture agent in solution, so that the method comprises contacting the micro-organisms with the capture reagent in solution and capturing said micro-organisms to a surface by binding said capture reagent to said surface by polar interaction between said surface and said capture reagent.

The sample may be a fluid sample such as sputum, urine, blood, bronchial lavage, etc. or may be a solid sample such as a tissue biopsy, e.g. a skin sample, which preferably is treated to extract or disperse micro-organisms into a liquid to produce a fluid sample.

pDADMAC comprises a long hydrocarbon chain bearing multiple polar, i.e. ionic sites spaced along said chain and is cationic. Since most bacterial cells are negatively charged the effect of p-DADMAC binding to the mycobacterial waxy coat is that the cells are converted to a net positive charge. This is advantageous as it ensures that other contaminating organisms that do not bind p-DADMAC remain negatively charged and so do not become bound to the micro-beads.

In addition, in direct capture embodiments, organisms that are not sufficiently hydrophobic will not bind to p-DADMAC coated surfaces in the presence of detergents, thus giving a degree of selectivity of the type of organism captured.

The capture reagent should preferably be sufficiently hydrophobic in character to bind hydrophobically to plastics, e.g. to the polystyrene microplates usually employed to bind proteins, or alternatively may be able to bind to glass or a glass like surface, either by polar interaction or by being sufficiently hydrophobic in character to bind hydrophobically to the surface, which may suitably be such as might be found in microscope slides or cover slips. But is should be sufficiently hydrophilic in character that it will be soluble in water or in buffered aqueous medium, at least in the presence of a suitable detergent system or a tolerable amount of an organic co-solvent such as DMSO. It is therefore soluble in the admixture with the sample and any other materials used.

Irrespective of the above theory, the invention provides in a second, independent aspect, a method for the capture from a fluid sample of micro-organisms having a hydrophobic surface, which method comprises contacting the micro-organisms with a soluble capture reagent which comprises poly-DADMAC whereby the capture reagent binds said micro-organisms, and capturing said micro-organisms to a surface by binding said capture reagent to said surface.

In either aspect of the invention, said surface is suitably provided by beads. These may be of micro or nano dimensions. Suitably they are paramagnetic for easy separation from liquid media. They may have a carboxylic acid polymer surface or a surface characterised by sulphate or phosphate groups.

The molecular weight of the poly-DADMAC may be in the range of less than 100,000 (very low), 100,000 - 200,000 (low), 200,000 - 400,000 or 500,000 (medium) or over 500,000 (high).

Preferably, the sample is contacted with the capture reagent in the presence of a detergent system of one or more detergents which enhances the selectivity of the binding of the desired micro-organisms. Desirably, the micro-organisms are bound without binding some or all of the contaminating hydrophobic materials present in the sample or without binding some or all of the micro-organisms in the sample which are not those whose capture is desired.

The detergent system may comprise an amino acid amide of a fatty acid which is preferably N-lauroyl sarcosine. The detergent system may alternatively or further comprise a Triton X detergent, preferably Triton X-100.

For most samples, the capture reagent is preferably provided in a capture buffer, suitably having a pH of from 7-10, more preferably 7-9, e.g. from 8-9 or 8.2-8.6, such as a phosphate buffer or a Tris buffer. With very thick, mucoid sputum samples that contain large quantities of mucopolysaccharides that have many carboxylic acid groups a lower pH for capture may be beneficial. At a sufficiently low pH the carboxyl groups are neutralized and do not interfere with pDADMAC binding of the mycobacteria and the subsequent capture of the pDADMAC. Such conditions, although designed to deal with highly mucoid samples may be employed with all samples. Suitably the pH of the capture reagent in this case is from 0 to 4, the pH of 4 being low enough still to protonate carboxylic acid groups. Thus, depending on the choice of solid surface, the pH of the capture reagent may at least be from 0 to 10.

The processing of the sample may of course include a decontamination stage in which the sample, or the surface bearing the captured micro-organisms is treated to render non-viable micro-organisms other than those of interest. This may be performed with materials known for the purpose such as sodium hydroxide with or without N-acetyl cysteine, or with N-acetyl cysteine alone. The aim is of course to leave the captured micro-organisms of interest in a viable state.

The captured micro-organism may in particular be a mycobacterium, which may be any of those referred to above.

The invention includes a method for the detection of a micro-organism, comprising capturing said micro-organism to a surface by a method as described, washing said captured micro-organism, and detecting said captured micro-organism on said surface or after removal therefrom.

The detection method used may be any appropriate to the micro-organism in question. For mycobacteria in general and *N. tuberculosis* in particular, these will include culturing and microscopic detection, e.g. by staining, PCR - polymerase chain reaction, TMA - transcription mediated amplification, SDA - strand displacement assay, or other amplification and detection methodologies directed to the nucleic acids of the organism itself, and phage based methods including FASTPlaqueTB where mycobacterium infecting phage is added and allowed to enter the cells, phage that is left outside the cells is killed and after further incubation to release phage from the cells, the presence of the released phage is detected by infecting a further micro-organism.

The materials, or selected key materials, needed for the practice of the micro-organism detection methods described above may be provided in kit form. Accordingly, the invention includes a Mycobacteria assay kit comprising either (a) a soluble capture reagent having both a hydrophobic character whereby the capture reagent is capable of binding Mycobacteria to be detected by hydrophobic interaction therewith and a polyionic character, said capture reagent being pDADMAC, a substrate having a surface for capturing said Mycobacteria to said surface by binding said capture reagent to said surface by polar interaction between said surface and said capture reagent, or (b) a capture reagent coated on and thus immobilised upon a solid surface, said capture reagent having both a hydrophobic and polyionic character whereby the capture reagent is capable of binding Mycobacteria to be detected, said capture reagent being pDADMAC,
and at least one of:
- phage capable of infecting said Mycobacteria;
- primers for carrying out an amplification of genomic nucleic acid of said Mycobacteria or said phage;
- an acid fast stain for visualising said Mycobacteria for microscopic inspection; or
- an antibody for binding said Mycobacteria;
and optionally further contains:
- a culture medium for culturing said Mycobacteria;
- a detection reagent for use in detecting a metabolite produced upon culture of said Mycobacteria;
- a detection agent specific for viable Mycobacteria;
and/or
- one or more drugs potentially able to affect the viability of said Mycobacteria.

In accordance with the invention described above, the sample may also be a gaseous, e.g. air, sample having micro-organisms entrained therein. Such a sample may be bubbled into the capture reagent solution to bind the micro-organisms to the capture reagent.

The solid surface may be a microscope slide.

Preferably, the captured mycobacteria, either captured directly or indirectly, are not harmed by this capture and remain viable. Thus the invention can be used for drug susceptibility testing of the organism. In one aspect, the mycobacteria can be exposed to a drug in such a way as to allow the drug to affect the organism. Subsequently, the mycobacteria can be captured in any of the ways described herein and then can be investigated for viability using any number of previously described methods which might include microscopy using viability stains, phage based methods, culture-based methods or PCR-based methods. In another aspect, the mycobacteria can be first captured in any of the ways described herein then subsequently exposed to a drug in such a way as to allow the drug to affect the organism. Subsequently, the mycobacteria can then be investigated for viability using any number of described methods which might include microscopy using viability stains, phase based methods, culture-based methods or PCR-based methods. The drugs used may include those commonly used to treat tuberculosis such as rifampicin, streptomycin, isoniazid, ethambutol, pyrazinamide, and ciprofloxacin.

M. tuberculosis is carried in airborne particles, the droplet nuclei, that are generated when infected subjects who have pulmonary or laryngeal TB disease cough, sneeze or shout. The particles are approximately 1-5 µm and can remain airborne for several hours, ensuring that they can spread throughout a room or building. Infection occurs when a susceptible person inhales the droplet nuclei containing M. tuberculosis, which then traverse the mouth or nasal passages, upper respiratory tract and bronchi to reach the alveoli. MDR M. tuberculosis is also classified by CDC as a category C agent of biological terrorism and the delivery mechanism is likely to be the generation of an airborne aerosol.

It is desirable to protect health workers, other persons in the vicinity of the infected subject and military personnel from the danger of infection by inhalation. In addition, laboratory staff who are working with TB-infected samples, TB cultures and samples containing other pathogenic mycobacteria (such as M. paratuberculosis in faeces) are also at risk from infection. Currently health workers and laboratory staff attempt to prevent infection using face masks, or a more sophisticated particulate-filter respirator.

The CDC recommends that a National Institute for Occupational Safety and Health (NIOSH)-certified particulate-filter respirator (e.g., N95, N99, or N100) should be used, with the ability to efficiently filter the smallest particles in the 1-5 µm range. Face masks are generally composed of simple woven or non-woven materials; they may have several layers and may have a specification that indicates a defined pore size. However, most masks are not NIOSH-certified as respirators, do not protect the user adequately from exposure to TB and do not satisfy OSHA requirements for respiratory protection. A study has shown that the use of respiratory protection is estimated to reduce the risk of infection in health care workers by the following proportions (compared to no protection): surgical face mask, 2.4-fold; disposable dust, fume, mist, or disposable high-efficiency particulate air filtering (HEPA) mask, 17.5-fold; elastomeric HEPA cartridge respirator, 45.5-fold; or powered air-purifying respirator (PAPR), 238-fold. (J Occup Environ Med. 1997 Sep;39(9):849-54).

Whilst the particulate-filter respirator provides a high level of protection it has the disadvantage of high cost and is restrictive in use. There is a need for an improved, disposable face mask that provides enhanced protection for the user from airborne mycobacteria infection in situations where the respirator is not available or is inappropriate to use. This is the situation in developing countries and also in the laboratory setting. What is required is a face mask and/or filter that provides a specific and efficient method of binding mycobacterium-containing aerosols generated by infected subjects and accidentally generated in the laboratory, so greatly improving the standard of user protection.

One can therefore provide a filter for filtering a gas stream to remove micro-organisms entrained therein, said filter comprising a polar surface and a capture reagent on or upstream of the polar surface, which capture reagent has both a hydrophobic character whereby it is capable of binding hydrophobically coated bacteria by hydrophobic interaction and a polar character, e.g. a polyionic character, whereby it is bound to or is adapted to bind to said polar surface.

The filter may take the form of a face mask for protecting a wearer or may be a filter unit attached to a face mask or helmet. It may be a filter installed or for installation in an air supply duct.

To provide improved protection, a face mask can be provided that is impregnated with a soluble capture reagent having both a hydrophobic character whereby the capture reagent binds mycobacteria by hydrophobic interaction and a polar character, e.g. polyionic character, whereby the capture reagent binds to an ionic surface by polar interaction.

The soluble capture agent can be sprayed onto a suitable solid phase mask material such as the filter material of the face mask and then dried prior to packaging of the product. When in use the face mask will become moist due to exhaled breath from the user and the capture agent will then become solubilised in the layer of moisture on the surface of the mask material. The impact of mycobacteria-containing aerosols to this surface will result in rapid binding of the soluble capture reagent to the mycobacteria cells. Use of a solid phase material in the mask that is polyionic will lead to immobilisation of the mycobacterium to the solid phase. This will eliminate any possibility of the further generation of an infectious aerosol from the surface during inhalation and will provide a high level of protection for the operator.

In this first example the soluble capture reagent will become bound to the polyionic solid phase material of the mask on wetting and prior to aerosol impact. This may have the effect of reducing efficiency of capture of the mycobacterium as the surface could become saturated with the capture reagent and so will not bind the mycobacterium cells/soluble capture reagent complex in the impacting aerosols. Alternatively, the bound capture reagent may have a reduced affinity of avidity for the micro-organisms by virtue of interference from the solid surface.

This disadvantage can be overcome by using a two layer face mask that has a first outer layer impregnated with the soluble capture reagent onto a neutral, uncharged material. Impacting aerosols will result in the formation of a mycobacterium/soluble capture reagent complex that then becomes tightly bound by the polyionic material in the second inner layer of the face mask structure.

Accordingly, one may have a filter as described initially, wherein said capture reagent is provided on a solid surface having low binding affinity for the capture reagent upstream of said polar surface.

In the accompanying drawings:
Figure 1 shows microscope visualisation of Ziehl Neelson staining of *Mycobacterium bovis* in Example 10 in step 5 (left hand panel) and after step 6 (right hand panel);
Figure 2 shows at higher (top panel) and lower (bottom panel) magnification the micro-organisms isolated from beads in step 6 of Example 10;
Figure 3 shows coated (left) and uncoated (right) processed in Example 11; and
Figure 4 shows mycobacteria captured in Example 12 and stained to demonstrate viability.

The invention will be further described and illustrated by the following Examples. In these examples, as *M. smegmatis* shares many properties in common with *M.tuberculosis* but is not infectious, it was used as a representative model organism for the mycobacterium genus.

### EXAMPLES

### Example 1. Titration of pDADMAC Ligand and Capture Beads

Rationale. This experiment was performed in order to determine the optimal quantity of ligand and beads to use for capture of the mycobacterium. The quantity of captured mycobacterium was analysed by PCR of the mycobacterium genome.

### Method

1. Replicates of 1µ1 of a culture of *Mycobacterium smegmatis* were made into 1 ml 7H9 OADC (7H9 media supplemented with 10% OADC, Difco) culture media.
2. 250µl of 5x Capture Buffer (250 mM Tris pH 8.3, 5%(w/v) N-lauroyl sarcosine, 5% (v/v) Triton X-100, 5% (w/v) BSA) was added and mixed.
3. Various quantities of pDADMAC (Sigma Aldrich, medium molecular weight, 400,000 to 500,000) diluted in water were added, mixed and incubated for 15 min.
4. MyOne Carboxylic Acid paramagnetic beads were added at a volume ratio of 10:1 compared to the initial volume of pDADMAC, mixed and incubated 15 min.
5. The beads were captured via a magnetic stand and washed in 1 ml PBS.
6.20µl 100mM NaOH, 0.05% (v/v) Triton X-100 was added and the beads resuspended and heated at 95°C for 5 min.
7. 10µl 200mM HCl was added and 2 µl of the eluate analysed by quantitative PCR for *Mycobacterium smegmatis.*

### PCR analysis.

An MJ Research Inc. (Hercules, California) Chromo 4 machine was used. Sybr Green kits (Eurogentec, Seraing, Belgium) were used which enables PCR product to be monitored through the fluorescence of the DNA double strand intercalator. PCR parameters used included, heating at 95°C for 10 sec, annealing primers at 65°C for 15 sec and extension at 72°C for 15 sec. PCR primers 5' TCA GGC CCT CGA AAG CCG ACT GGG 3', 5' CCA GGA CTC GGT ACA AGA CTC TGC 3' specific for the *M. smegmatis* genome were used.

### Results

| Quantity of pDADMAC used | Quantity of beads used | Cycle at which PCR was positive. *M. smegmatis* present | Cycle at which PCR was positive. No *M. smegmatis* control |
|---|---|---|---|
| 5µl 0.01% (v/v) | 50µl | 25.2 | 34.1 , |
| 2µl 0.01% (v/v) | 20µl | 27.2 | Remained negative |
| 5µl 0.004% (v/v) | 10µl | 26.5 | 37.1 |
| 2.5µl 0.004% (v/v) | 2.5µl | 28.7 | 35.7 |

### Conclusion

5µl 0.01% pDADMAC worked best, giving a signal at cycle 25 compared to cycle 34 for the no-bacilli control (PCR primer-dimer background). Dilutions of pDADMAC and beads gave a progressively reduced recovery of *M. smegmatis.*

### Example 2. Investigation of the efficiency of capture.

The efficiency of capture of *M. smegmatis* spiked into media was investigated compared to the same quantity of *M. smegmatis* extracted by alkali heating and detected by PCR directly.

### Method

1. Dilutions of a culture of *Mycobacterium smegmatis* were made into 1 ml 7H9 OADC (7H9 media supplemented with 10% OADC, Difco) culture media.
2.250µl of 5x Capture Buffer (250 mM Tris pH 8.3, 5%(w/v) N-lauroyl sarcosine, 5% (v/v) Triton X-100, 5% (w/v) BSA) was added and mixed.
3. 10µl of 0.01% (v/v) pDADMAC (Sigma Aldrich, medium molecular weight, 400,000 to 500,000) was added, mixed and incubated for 15 min.
4. 50µl MyOne Carboxylic Acid paramagnetic beads were added, mixed and incubated 15 min.
5. The beads were captured via a magnetic stand and washed in 1 ml PBS.
6. 20µl 100mM NaOH, 0.05% (v/v) Triton X-100 was added and the beads resuspended and heated at 95°C for 5 min.
7. 10µl 200mM HCl was added and 2 µl of the eluate analysed by quantitative PCR for *Mycobacterium smegmatis.*
8. In addition, 1 µl of the same *M. smegmatis* culture was treated directly with alkali as described in steps 6-7 above and analysed by PCR in the same way.

### PCR analysis.

The PCR is described in example 1.

### Results

| Dilution of *M. smegmatis* | Approx. number of bacilli | Cycle at which PCR was positive |
|---|---|---|
| 10⁻³ | 100,000 | 26.7 |
| 10⁻⁴ | 10,000 | 32.0 |
| 10⁻⁵ | 1000 | 37.3 |
| No *M. smegmatis* control | 0 | 35.6 |
| 1µl *M. smegmatis* treated directly | 100,000 | 26.5 |

### Conclusion

The efficiency of capture of the bacilli was very high with a similar signal generated from the same quantity of bacilli spiked and recovered as analysed directly. As few as 10,000 bacilli spiked into the ml of media could be recovered and detected.

### Example 3. Investigation of the requirement for capture buffer.

Rationale. *M. smegmatis* spiked into media was recovered in the presence or absence of capture buffer.

### Method.

The method was as described in example 1 except that in one sample no capture buffer was added.

### Results

| Capture buffer present | Cycle at which the PCR was positive |
|---|---|
| No capture buffer | 25.5 |
| Capture buffer used | 23.0 |

### Conclusion

The capture buffer enhanced recovery of the bacilli by 2.5 cycles or about 6-fold in terms of bacilli genomes and bacilli. This is probably due to the action of the detergents on the media and reduced interference by inhibitory elements that inhibit binding of the *M. smegmatis* to the capture reagent.

### Example 4. Demonstration of the utility of the ligand capture of M. smegmatis in a phage based assay.

Rationale. Mycobacteria can be tested for viability via the ability of the bacteria to host bacteriophage infection. One of the problems of this approach is to separate the infected bacilli from the exogenous non-infecting bacteriophage. Once separated from exogenous phage the bacilli can be lysed and investigated for endogenous, infecting bacteriophage.

### Method.

100µl of *M. smegmatis* was added to 10 ml 7H9 OADC media and incubated for 3 hours at 37°C. A negative control without bacilli was also prepared.

100µl (about 10¹⁰ pfus) D29 mycobacteriophage were added to both tubes and the samples placed back in the incubator.

1ml aliquots were removed at various time points post-infection and the *M. smegmatis* captured from the media as described in example 1, except that three additional washes were performed in PBS.

The captured bacilli were lysed as described and investigated for the presence of endogenous infecting phage genome by PCR. The PCR was as described previously except that phage genome specific primers 5' CCT CGG GCT AAA AAC CAC CTC TGA CC 3', 5' CTG GGA GAA TGT GAC ACG CCG ACC 3' were used.

### Results

| Time post infection | *M. smegmatis* present | Cycle at which PCR was positive |
|---|---|---|
| 15 min | Yes | Remained negative |
| 15 min | No | 39.8 |
| 30 min | Yes | 27 |
| 30 min | No | Remained negative |
| 60 min | Yes | 30 |
| 60 min | No | Remained negative |
| 90 min | Yes | Remained negative |
| 90 min | No | Remained negative |
| 120 min | Yes | 31 |
| 120 min | No | Remained negative |

### Conclusion

The ability to capture *M. smegmatis* from the media allowed the bacilli to be washed and exogenous phage to be removed. The only phage that were subsequently detected were those that had infected the bacilli. In this example the process has been used to monitor the infection process. 15 min after addition of the phage there was no signal detected from the bacilli. The phage have yet to infect and the phage genome is not replicated. Endogenous phage genome appears at 30 min in the bacilli but declines at time point 60 min, disappearing completely at time point 90 min as the phage replicate and lyse the bacilli. The signal reappears at 120 min as the released second generation replicated phage undergo another round of infection and replication.

### Example 5. Demonstration of the capture of mycobacteria from sputum

Rationale. Sputum is a complex and viscous matrix. The experiment was performed to show that this matrix would not interfere with the capture of mycobacteria.

### Method.

A pool of 5 sputum samples was prepared and aliquotted into 1 ml volumes.

10µl of *M. smegmatis* culture was added to half the aliquots.

100µl of 5M NaOH, 2.5% N-acetyl cysteine was added and incubated for 15 min to thin and decontaminate the sputum.

100µl of 5M HCl was added followed by 250µl 5x Capture Buffer (as described previously).

The *M. smegmatis* was then captured from the sputum and quantitated by PCR as described in example 2, steps 3-8.

### Results

The sample with *M. smegmatis* was positive at PCR cycle 20. The sample without bacilli (i.e. background) was positive at PCR cycle 36.3.

### Conclusion

The extraction method using pDADMAC and bead capture was not inhibited by the sample matrix (sputum) and *M. smegmatis* was successfully recovered from that sample.

### Example 6. Demonstration of the capture of mycobacteria from sputum without the requirement for thinning and decontamination with alkali.

Rationale. Sputum is a complex and viscous matrix. Treatment with alkali thins and decontaminates the sputum but can also damage the mycobacteria. This experiment was performed to show that the extraction procedure can be used without prior alkali treatment. Again, *M. smegmatis* was used as a model organism for the mycobacteria genus.

### Method.

A pool of 5 sputum samples was prepared and aliquotted into 1 ml volumes.

10µl of *M. smegmatis* culture was added to half the aliquots.

250µl 5x Capture Buffer (as described previously) was added and mixed.

The *M. smegmatis* was then captured from the sputum and quantitated by PCR as described in example 2, steps 3-8.

### Results

The sample with *M. smegmatis* was positive at PCR cycle 24.7. The sample without bacilli (i.e. background) remained negative.

### Conclusion

The extraction method using pDADMAC and bead capture did not require prior treatment of the sputum with alkali. It was observed that the addition of capture buffer was sufficient to cause the breakdown and thinning of the sputum which subsequently allowed the recovery of *M. smegmatis* from that sample.

### Example 7. Demonstration of the requirement for the pDADMAC ligand in the capture system.

Rationale. This experiment was performed in order to demonstrate that the capture reagent, exemplified here by pDADMAC, is crucial for capture of mycobacteria and that the mycobacteria do not bind to the carboxyl bead in the absence of capture reagent.

### Method.

0.5 ml aliquots of a stationary phase culture of *M. smegmatis* were centrifuged to pellet the organism then the organisms resuspended in Capture Buffer (50mM Tris pH 8.3, 1% (w/v) N-lauroyl sarcosine, 1% (v/v) Triton X-100, 1% (w/v) BSA).

To one aliquot 10 µl 0.01% pDADMAC was added, mixed and incubated for 15 min. An identical aliquot had no pDADMAC added and was left for 15 min.

50µl MyOne carboxylic acid paramagnetic beads (washed x3 before use in dH₂O and resuspended in the original volume of dH₂O) were then added to each aliquot and incubated 15 min.

The aliquots were then placed on a magnet and any clearing of the turbid suspension of organisms assessed by eye.

The supernatants were then removed and kept.

The beads were then washed in x1 Capture buffer and x2 in 7H9 OADC media and resuspended in 1 ml of this media.

An equivalent of 0.1µl of the supernatant and the bead suspension were plated out on 7H9 OADC agar plates and incubated for 2 days at 37°C after which time the numbers of colonies on each plate were counted.

### Results

After addition of the magnetic beads and placing the aliquots on a magnet there was substantial clearing of the aliquot which had been incubated with the pDADMAC capture reagent. This was due to the capture of most of the organisms in the suspension onto the magnetic particles. The aliquot which did not have the capture reagent added remained turbid as the organisms remained in suspension. The numbers of captured and non-captured bacilli in the presence and absence of capture reagent were counted from the agar plate cultures and tabulated (see table below).

| | Number of colonies counted pDADMAC capture reagent absent | Number of colonies counted pDADMAC capture reagent added |
|---|---|---|
| Supernatant | Greater than 1000 | 490 |
| Bead captured | 359 | Greater than 1000 |

### Conclusion

The capture of the mycobacteria, as determined via visual turbidity, via the carboxylic acid beads was dependent on the presence of the pDADMAC. This visual observation was confirmed by microbiological quantitation. In the presence of capture reagent the vast majority of the mycobacteria were captured whereas in the absence of capture reagent there was minimal adsorption to the beads.

### Example 8. Demonstration of the selectivity of the ligand capture for mycobacteria.

Rationale. This experiment was performed in order to demonstrate that the pDADMAC capture reagent binds specifically to mycobacteria and does not bind to other tested organisms, including representative gram negative and gram positive organisms, that may also contaminate relevant biological specimens.

### Method.

0.5 ml aliquots of stationary phase cultures of *M. smegmatis, Pseudomonas aeruginosa, Staphylococcus aureus* and *Escherichia coli* were centrifuged to pellet the organisms then the organisms resuspended in Capture Buffer (50mM Tris pH 8.3, 1% (w/v) N-lauroyl sarcosine, 1% (v/v) Triton X-100, 1% (w/v) BSA).

To each aliquot of each organism 10 µl 0.01% pDADMAC was added, mixed and incubated for 15 min.

50µl MyOne carboxylic acid paramagnetic beads (washed x3 before use in dH₂O and resuspended in the original volume of dH₂O) were then added to each aliquot and incubated 15 min.

The aliquots were then placed on a magnet and any cleaving of the turbid suspension of organisms assessed by eye.

The supernatants were then removed and kept.

The beads were then washed in x1 Capture buffer. The *M. smegmatis* aliquots were then washed x2 in 7H9 OADC media and resuspended in 1 ml of this media. The other organisms were washed the same way in Mueller Hinton medium and resuspended in 1 ml of this media.

An equivalent of 0.1µl of the supernatant and the bead suspension were plated out on either 7H9 OADC agar plates for *M. smegmatis* or Mueller Hinton agar plates for the other organisms and incubated for at 37°C until bacterial colonies appeared after which time the numbers of colonies on each plate were counted.

### Results

As before, the suspension of *M. smegmatis* was cleared when placed on the magnet demonstrating that the organism had been captured from solution. The suspensions of all other organisms tested remained very turbid demonstrating that the the organisms were not captured and remained in suspension. The numbers of captured and non-captured bacilli were counted from the agar plate cultures and tabulated.

| Organism | Number of colonies from the supernatant | Number of colonies from the beads |
|---|---|---|
| *M. smegmatis* | 221 | Greater than 1000 |
| *P.aeruginosa* | Greater than 10,000 | 12 |
| *S.aureus* | Greater than 1000 | 8 |
| *E.coli* | Greater than 10, 000 | 22 |

### Conclusion

The pDADMAC capture reagent allowed the specific capture of the mycobacterium *M. smegmatis.* The other organisms tested did not bind to this capture reagent and were not captured.

### Example 9. Investigation of the capture buffer composition required for specific capture.

Rationale. This experiment was performed in order to investigate the components of capture buffer that are important in the specific binding of pDADMAC to mycobacteria.

### Method.

0.5 ml aliquots of stationary phase cultures of *M. smegmatis, Pseudomonas aeruginosa, Staphylococcus aureus* and *Escherichia coli* were centrifuged to pellet the organisms then the organisms resuspended in various component parts of the capture buffer.

To each aliquot of each organism 10 µl 0.01% pDADMAC was added, mixed and incubated for 15 min. To another aliquot of each organism, no capture reagent was added as a control for observation of capture reagent mediated capture.

50µl MyOne carboxylic acid paramagnetic beads (washed x3 before use in dH₂O and resuspended in the original volume of dH₂O) were then added to each aliquot and incubated 15 min. The aliquots were then placed on a magnet and any clearing of the turbid suspension of organisms assessed by eye.

### Results

The results were recorded and presented in the table below. The non mycobacteria organisms were not captured by the beads in the absence or presence of capture reagent under any buffer conditions. The capture of the mycobacteria was dependent on the presence of the pDADMAC capture reagent and capture was observed under all buffer conditions studied. The use of a buffer containing N-lauroyl sarcosine only, appeared to partially inhibit the capture. When sarcosine was used in combination with Triton-X100, however the efficiency of capture was restored. If N-lauroyl sarcosine was not present in the capture buffer the beads were very clumped after resuspension after mycobacterial capture. Inclusion of N-lauroyl sarcosine prevented this post-capture clumping and aided the manipulation of the beads which would be important for subsequent bead washing and post-capture processing and analysis.

### Conclusion

As demonstrated previously, non-mycobacterial organisms were not captured to the beads in the presence or absence of capture reagent under any conditions tested. The capture of mycobacteria was dependent on the presence of the capture reagent and could be demonstrated in all conditions tested. The inclusion of N-lauroyl sarcosine in the capture buffer was crucial for post-capture manipulation and analysis of the captured material.

### Example 9: Results Table

| | **Organism used** | | | | | | | |
|---|---|---|---|---|---|---|---|---|
| **Capture buffer used** | *P.aeruginosa* With Capture reagent | *P.aeruginosa* Without Capture reagent | *S.aureus* With Capture reagent | *S.aureus* Without Capture reagent | *E.coli* With Capture reagent | *E.coli* Without Capture reagent | *M. smegmatis* With Capture reagent | *M. smegmatis* Without Capture reagent |
| 50 mM Tris pH 8.4 | Remained turbid | Remained turbid | Remained turbid | Remained turbid | Remained turbid | Remained turbid | Clear | Remained turbid |
| 1% N-lauroyl sarcosine | | | | | | | | |
| 1% (v/v)Triton X-100 | | | | | | | | |
| 50 mM Tris pH 8.4 | Remained turbid | Remained turbid | Remained turbid | Remained turbid | Remained turbid | Remained turbid | Slightly turbid | Remained turbid |
| 1% N-lauroyl sarcosine | | | | | | | | |
| 50 mM Tris pH 8.4 | Remained turbid | Remained turbid | Remained turbid | Remained turbid | Remained turbid | Remained turbid | Clear | Remained turbid |
| 1% (v/v) Triton X-100 | | | | | | | | |
| 50 mM Tris pH 8.4 | Remained turbid | Remained turbid | Remained turbid | Remained turbid | Remained turbid | Remained turbid | Clear | Remained turbid |

### Example 10. Detection of mycobacteria by ligand capture and acid-fast microscopy.

*Mycobacterium bovis* was captured from solution and stained by both acid fast Ziehl Neelsen colour stain and auramine phenol fluorescent stain directly on the bead or after elution.

### Method

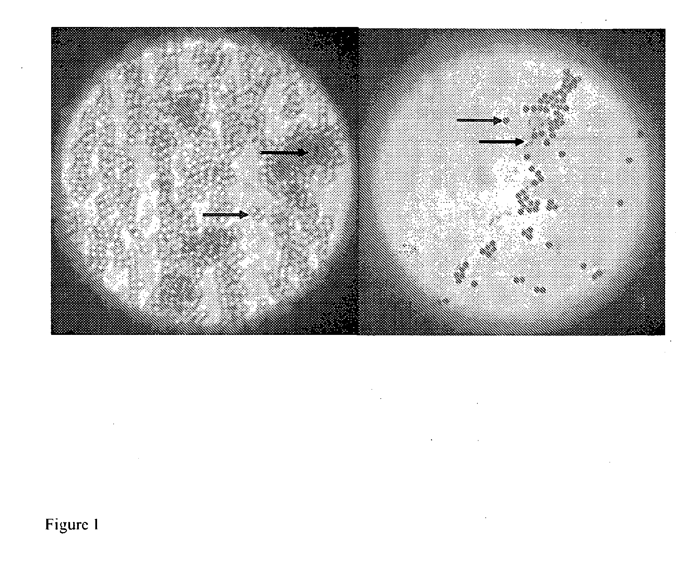

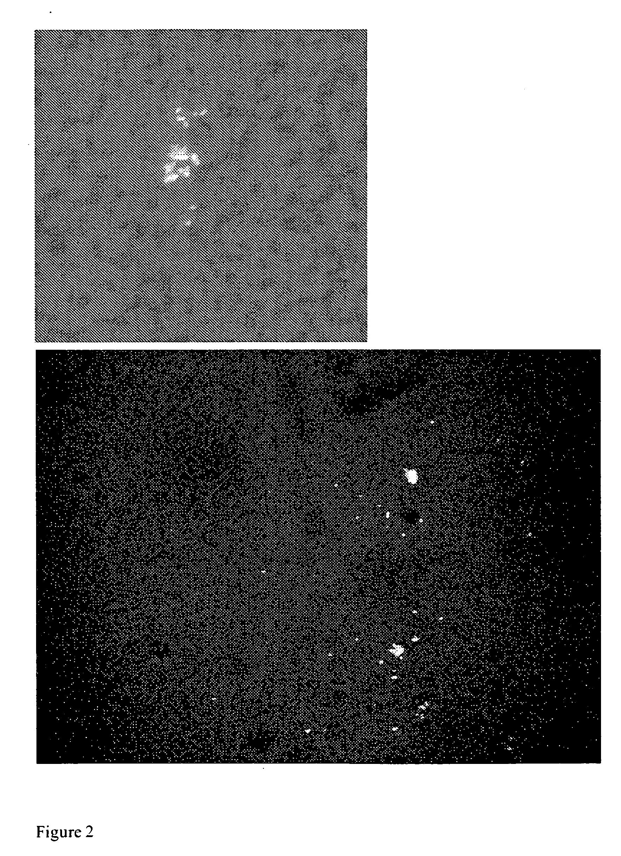

1. 250µl of 5x Capture Buffer (250 mM Tris pH 8.3, 5% (v/v) Triton X-100, 5% Zwittergent [3-(n,N-dimethylmyristylammonia)-propanesulfonate]) was added to 1 ml of 7H9 medium containing a suspension of *Mycobacterium bovis.*
2. 10µl of 0.01%(v/v) pDADMAC was added, mixed and incubated for 15 min.
3. 50µl MyOne Carboxylic Acid paramagnetic beads were added, mixed and incubated 15 min.
4. The beads were captured via a magnetic stand and washed in 1 ml PBS.
5. Half the volume of beads were spotted onto a microscope slide, dried and heat fixed prior to Ziehl Neelsen staining. In Figure 1 left hand panel, captured mycobacteria are seen prior to elution from the ligand/beads. The magnetic beads are indicated by the lower arrow. Bead-captured, highly aggregated acid fast mycobacteria are indicated by the upper arrow and can be seen surrounded by beads.
6. The remaining beads were resuspended in 100µl dH2O and 10µl chloroform added. After vortexing to mix the chloroform with the aqueous layer, the beads were pulled to the side of the tube with a magnet and the supernatant spotted onto a slide, dried, heat fixed and stained by both Ziehl Neelsen staining and auramine phenol. All staining was performed as described in Medical Microbiology, a Practical Approach, Eds., Peter Hawkey and Deidre Lewis, Oxford University Press. In Figure 1, right hand panel, captured mycobacteria are seen after elution from the ligand/beads. The elution has dispersed the acid fast mycobacteria (lower arrow). Some beads are still present (upper arrow). Figure 2 shows the micro-organisms captured from the beads. At high magnification in the upper panel a clump of ligand-captured fluorescent mycobacteria can be seen and in the lower panel at lower magnification the typical 'starry night' of dispersed mycobacterial fluorescence can be seen.

### Results

The mycobacteria are captured by the ligand/paramagnetic beads and, without elution, after Ziehl Neelsen staining can be seen as a highly aggregated pink material surrounded by beads. After elution, the mycobacteria are separated from the beads and are dispersed (see Figures 1 and 2).

### Conclusion

The mycobacteria can be captured by the TB-ligand and can be visualised by acid fast staining and microscopy. After elution, the mycobacteria are isolated from the beads and are dispersed. Further experiments have demonstrated that the ligand capture and staining protocol works well for clinical TB samples in sputum and that fluorescent microscopy can be used for a more sensitive detection.

### Example 11. Direct capture of mycobacteria on ligand coated solid surface with in situ staining and detection of captured organisms by microscopy.

Rationale. This experiment was performed in order to demonstrate the capture of mycobacteria to p-DADMAC coated slides visualised by in situ staining and microscopy.

### Method.

A microscope slide was coated with p-DADMAC by flooding the slide with 2%(v/v)p-DADMAC (diluted from a 20% stock in distilled water) and allowing it to evapourate to dryness. An uncoated slide was used as a control. The slides were then washed in copious amounts of distilled water and dried. 100µl of *M. smegmatis* culture was added to 800µl dH2O and 100µl Capture Buffer (10%(w/v) Zwittergent [3-(n,N-dimethylmyristylammonia)-propanesulfonate], 10% (v/v) Triton X-100, 500mM Tris pH 8.3)and spotted onto the slides. After incubation for 10 min the slides were washed in distilled water and gram stained as described in Medical Microbiology, a Practical Approach, Eds., Peter Hawkey and Deidre Lewis, Oxford University Press.

### Results

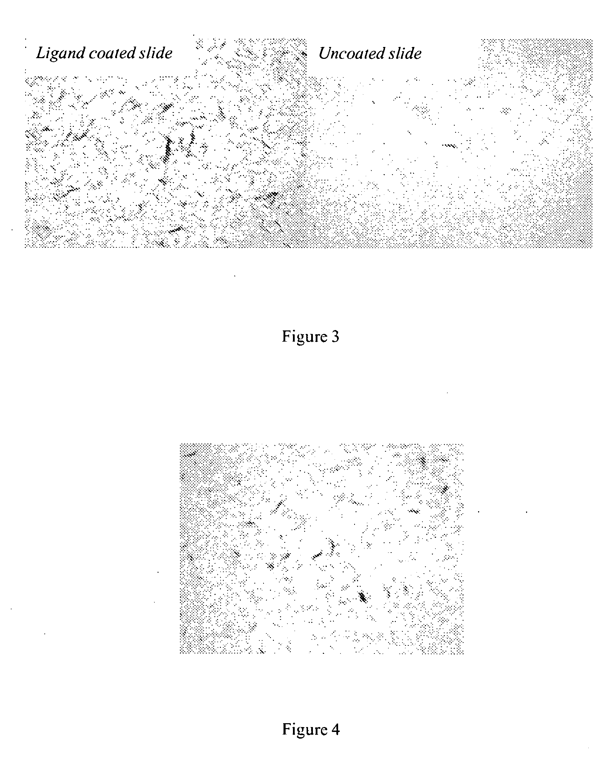

Gram positive mycobateria could be observed by microscopy captured in large numbers onto the p-DADMAC coated slide (see Figure 3) whereas very few (if any) mycobacteria were captured on the uncoated slide.

### Conclusion

This demonstrates that mycobacteria can be captured by the p-DADMAC coated slide and that these mycobacteria can be stained in situ and observed by microscopy. Similar results were also obtained from cultures of BCG and staining by acid fast Ziehl Neelsen stain and fluorescent auramine phenol stain.

### Example 12. Direct capture of mycobacteria on ligand coated solid surface with in situ viability staining and detection by microscopy.

Rationale. This experiment was performed in order to demonstrate that the mycobacteria captured to p-DADMAC coated slides remain viable and could be visualised by in situ viability stains followed by microscopy.

### Method.

A microscope slide was coated with p-DADMAC by flooding the slide with 2%(v/v)p-DADMAC (diluted from a 20% stock in distilled water) and allowing it to evapourate to dryness. An uncoated slide was used as a control. The slides were then washed in copious amounts of distilled water and dried. 100µl of *M. smegmatis* culture was added to 800µl dH2O and 100µl Capture Buffer (10%(w/v) Zwittergent [3-(n,N-dimethylmyristylammonia)-propanesulfonate], 10% (v/v) Triton X-100, 500mM Tris pH 8.3)and spotted onto the slides. After incubation for 10 min the slides were washed in distilled water and 1mg/ml Thiazolyl Blue Tetrazolium Bromide(MTT) in 7H9, OADC media added and incubated for 30 min at room temperature. After washing in distilled water the viable mycobacteria were observed by microscopy.

### Results

The MTT stain is deposited as an insoluble blue/black stain in the viable organisms captured onto the p-DADMAC coated slide allowing the viable organisms to be detected by microscopy (see figure 4).

### Conclusion

This demonstrates that mycobacteria can be captured by the p-DADMAC coated slide and that these captured mycobacteria remain viable and can be stained by viability stains such as MTT.

### Example 13. Method for use with mucoid sputum

Rationale. Some sputum samples may be very thick and mucoid with a high concentration of mucopolysaccharides that are highly cross-linked by covalent sulphide bridges and highly charged with many carboxyl groups. The use of reducing agents such as dithiothreitol and N-acetyl cysteine to break the disulphide bonds has been discussed but the mucopolysaccharides, at high concentration, may still interfere with the capture of mycobacteria through the interaction of the negatively charged carboxyl groups with the positively charged pDADMAC. In order to reduce this inhibition it may be advisable to carry out the capture at a low pH - at a pH at which the carboxyl groups are neutralised but the pDADMAC remains charged. At such low pHs it would not be possible to capture the pDADMAC on carboxyl beads as these too would have lost their charge thus, at low pH carboxyl beads must be replaced with sulphate beads that remain negatively charged under conditions that carboxyl beads become neutral. These conditions were tested for the capture of *Mycobacterium tuberculosis* from sputum supplied by the World Health Organization sputum bank.

### Method

1. BioMag amine beads (BM546, Bangs Laboratories Inc., US) were first coated in 5% (v/v) pDADMAC (high molecular weight) in dH₂0 for 1 hour then, after washing in dH₂0 over-coated with 10 mg/ml dextran sulphate (500 000 mwt) for 1 hour in dH₂0. After washing in dH₂0 the beads were resuspended in the original volume of dH₂0 and were then ready for use.
2. 0.5 ml of purulent sputum samples (either microscopy positive or microscopy negative for mycobacteria) were treated for 20 min with 2% (w/v) final of dithiothreitol. As a positive control some sputum samples were also spiked with cultured BCG prior to treatment.
3.After this treatment, 50µl 10% (v/v) Triton X-100, 10mM EDTA, 20 µl 0.004% (v/v) pDADMAC (500 000 mwt)and 50µl 2.5M HCl was added and incubated for 10 min. The pH at this stage is expected to have been approximately 0.6.
4. 20µl of dextran sulphate-coated paramagnetic beads were then added and incubated for 10 min.
5. The beads were collected by magnet, washed in 1 ml dH20 (this washing step would not normally be required but was performed in order to demonstrate active capture of the mycobacteria), resuspended in 10µl dH₂0 and spotted onto a microscope slide.

The slides were processed for auramine phenol fluorescent microscopy of mycobacteria as described in example 10.

### Results

Ten sputum samples reported by the WHO sputum bank as negative for Mycobacterium tuberculosis were negative after capture and microscopy. Ten sputum samples reported by the WHO sputum bank as positive were clearly positive as were the controls spiked with BCG. Furthermore, the control samples indicated that there was a high efficiency of recovery of the mycobacteria from the sputum as it was estimated by comparative microscopy that 90-95% of the spiked mycobacteria were recovered.

### Conclusion

For thick, mucoid samples capture of mycobacteria worked well at a pH that was low enough to render the carboxylic acid groups on the mucopolysaccharides neutral.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

## Claims

1. A method for the capture from a sample of micro-organisms having a hydrophobic surface, said micro-organisms being Mycobacteria, which method comprises contacting the micro-organisms with a capture reagent, which capture reagent has both a hydrophobic character whereby the capture reagent binds said Mycobacteria by hydrophobic interaction therewith and a polar character and which is poly-diallyldimethyl ammonium chloride (pDADMAC), said capture reagent either being present on a surface and capturing said Mycobacteria thereto, or being present in solution, said method then further comprising capturing said Mycobacteria to a surface by binding said capture reagent to said surface by polar interaction between said surface and said capture reagent.

2. A method as claimed in claim 1, wherein said capture reagent comprises a long hydrocarbon chain bearing multiple polar sites spaced along said chain.

3. A method as claimed in claim 1 or claim 2, wherein said capture reagent is cationic.

4. A method as claimed in claim 3, wherein said poly-diallyldimethyl ammonium chloride (pDADMAC) has a molecular weight of from 200,000 to 500,000.

5. A method as claimed in Claim 1, which method comprises contacting the Mycobacteria with a soluble capture reagent which comprises poly-DADMAC whereby the capture reagent binds said Mycobacteria, and capturing said Mycobacteria to a surface by binding said capture reagent to said surface.

6. A method as claimed in any preceding claim, wherein the sample is contacted with the capture reagent in the presence of a detergent which enhances the selectivity of the binding of the desired micro-organisms.

7. A method as claimed in claim 6, wherein the detergent comprises an amino acid amide of a fatty acid.

8. A method as claimed in claim 6, wherein the detergent comprises N-lauroyl sarcosine.

9. A method as claimed in any one of claims 6 to 8, wherein the detergent comprises a Triton X detergent.

10. A method for the detection of a micro-organism, comprising capturing said micro-organism to a surface by a method as claimed in any preceding claim, washing said captured micro-organism, and detecting said captured micro-organism on said surface or after removal therefrom.

11. A method as claimed in claim 10, wherein the viability of the captured micro-organism is determined.

12. A method as claimed in claim 11, wherein the captured micro-organism is treated with a drug and the viability of the micro-organism is determined to establish whether the drug affects the viability of the micro-organism.

13. A Mycobacteria assay kit comprising either (a) a soluble capture reagent having both a hydrophobic character whereby the capture reagent is capable of binding Mycobacteria to be detected by hydrophobic interaction therewith and a polyionic character, said capture reagent being pDADMAC, a substrate having a surface for capturing said Mycobacteria to said surface by binding said capture reagent to said surface by polar interaction between said surface and said capture reagent, or (b) a capture reagent coated on and thus immobilised upon a solid surface, said capture reagent having both a hydrophobic and polyionic character whereby the capture reagent is capable of binding Mycobacteria to be detected, said capture reagent being pDADMAC,
and at least one of:
- phage capable of infecting said Mycobacteria;
- primers for carrying out an amplification of genomic nucleic acid of said Mycobacteria or said phage;
- an acid fast stain for visualising said Mycobacteria for microscopic inspection; or
- an antibody for binding said Mycobacteria;
and optionally further contains:
- a culture medium for culturing said Mycobacteria,
- a detection reagent for use in detecting a metabolite produced upon culture of said Mycobacteria,
- a detection agent specific for viable Mycobacteria,
and/or
- one or more drugs potentially able to affect the viability of said Mycobacteria.

14. A kit as claimed in claim 13, wherein said capture reagent is pDADMAC having a molecular weight of from 200,000 to 500,000.

15. A kit as claimed in claim 13 or claim 14, wherein said phage, said primers, said antibody or said detection agent is specific for the identification of *M. tuberculosis, M. avium, M. intracellulare, M. paratuberculosis, M. leprae, M. kansasii, M. marinum,* or *M. fortuitum* complex.

## Patentansprüche

1. Verfahren zum Einfangen von Mikroorganismen mit einer hydrophoben Oberfläche aus einer Probe, wobei die Mikroorganismen Mykobakterien sind, welches Verfahren das Inkontaktbringen der Mikroorganismen mit einem Fängerreagenz umfasst, welches Fängerreagenz ebenfalls einen hydrophoben Charakter aufweist, wobei das Fängerreagenz die Mykobakterien durch hydrophobe Wechselwirkung mit diesen und einem polaren Charakter bindet und ein Polydiallyldimethylammoniumchlorid (pDADMAC) ist, wobei das Fängerreagenz entweder auf einer Oberfläche vorliegt und die Mykobakterien auf dieser Oberfläche einfängt, oder in Lösung vorliegt, wobei das Verfahren dann ferner das Einfangen der Mykobakterien auf einer Oberfläche durch Bindung des Fängerreagenz an der Oberfläche durch polare Wechselwirkung zwischen der Oberfläche und dem Fängerreagenz umfasst.

2. Verfahren nach Anspruch 1, wobei das Fängerreagenz eine lange Kohlenwasserstoffkette enthält, die Träger mehrerer polarer Stellen ist, die entlang der Kette voneinander beabstandet sind.

3. Verfahren nach Anspruch 1 oder 2, wobei das Fängerreagenz kationisch ist.

4. Verfahren nach Anspruch 3, wobei das Polydiallyldimethylammoniumchlorid (pDADMAC) ein Molargewicht zwischen 200.000 bis 500.000 aufweist.

5. Verfahren nach Anspruch 1, wobei das Verfahren das Inkontaktbringen der Mykobakterien mit einem löslichen Fängerreagenz umfasst, das Poly-DADMAC enthält, wobei das Fängerreagenz die Mykobakterien bindet, und das Einfangen der Mykobakterien an einer Oberfläche durch Binden des Fängerreagenz an der Oberfläche.

6. Verfahren nach einem der vorangehenden Ansprüche, wobei die Probe in Gegenwart eines Detergens, das die Selektivität der Bindung der gewünschten Mikroorganismen erhöht, mit dem Fängerreagenz in Kontakt gebracht wird.

7. Verfahren nach Anspruch 6, wobei das Detergens ein Aminosäureamid von einer Fettsäure enthält.

8. Verfahren nach Anspruch 6, wobei das Detergens N-Lauroylsarcosin enthält.

9. Verfahren nach einem der Ansprüche 6 bis 8, wobei das Detergens ein Triton-X-Detergens enthält.

10. Verfahren zum Nachweis eines Mikroorganismus, umfassend:
Einfangen des Mikroorganismus an einer Oberfläche durch ein Verfahren nach einem der vorangehenden Ansprüche, Waschung des eingefangenen Mikroorganismus und Nachweis des eingefangenen Mikroorganismus auf der Oberfläche oder nach Entnahme von der Oberfläche.

11. Verfahren nach Anspruch 10, wobei die Lebensfähigkeit des eingefangenen Mikroorganismus bestimmt wird.

12. Verfahren nach Anspruch 11, wobei der eingefangene Mikroorganismus mit einem Pharmakon behandelt und die Lebensfähigkeit des Mikroorganismus bestimmt wird, um nachzuweisen, ob das Pharmakon die Lebensfähigkeit des Mikroorganismus beeinflusst.

13. Mykobakterium-Testkit, umfassend entweder (a) ein lösliches Fängerreagenz, das sowohl einen hydrophoben Charakter, wobei das Fängerreagenz in der Lage ist, Mykobakterien, die durch hydrophobe Wechselwirkung mit diesen nachgewiesen werden, zu binden, als auch einen polyionischen Charakter aufweist, wobei das Fängerreagenz pDADMAC ist, ein Substrat mit einer Oberfläche zum Einfangen der Mykobakterien an der Oberfläche durch Bindung des Fängerreagenz an der Oberfläche durch polare Wechselwirkung zwischen der Oberfläche und dem Fängerreagenz ist, oder (b) ein Fängerreagenz, mit dem eine feste Oberfläche beschichtet ist und welches somit auf einer festen Oberfläche immobilisiert ist, wobei das Fängerreagenz sowohl einen hydrophoben als auch einen polyionischen Charakter aufweist, wobei das Fängerreagenz in der Lage ist, nachzuweisende Mykobakterien zu binden, wobei das Fängerreagenz pDADMAC ist,
und umfassend zumindest:
- einen Phagen, der in der Lage ist, die Mykobakterien zu infizieren,
- Primer zur Durchführung einer Amplifikation der genomischen Nukleinsäure der Mykobakterien oder des Phagen,
- eine Säureschnellfärbung zur Sichtbarmachung der Mykobakterien zur mikroskopischen Untersuchung, oder
- einen Antikörper zum Binden der Mykobakterien,
und optional ferner umfassend:
- ein Kultivierungsmedium zur Kultivierung der Mykobakterien,
- ein Nachweisreagenz zur Verwendung beim Nachweis eines Metaboliten, der bei der Kultivierung der Mykobakterien entsteht,
- ein Nachweismittel zum spezifischen Nachweis von lebensfähigen Mykobakterien, und/oder
- ein oder mehrere Pharmaka, die potentiell in der Lage sind, die Lebensfähigkeit der Mykobakterien zu beeinflussen.

14. Kit nach Anspruch 13, wobei das Fängerreagenz pDADMAC ist, das ein Molargewicht zwischen 200.000 bis 500.000 aufweist.

15. Kit nach Anspruch 13 oder 14, wobei der Phage, die Primer, der Antikörper oder das Nachweismittel spezifisch für den Nachweis von *M. tuberculosis, M. avium, M. intracellulare, M. paratuberculosis, M. leprae, M. kansasii, M. marinum,* oder *M. fortuitum complex* sind.

## Revendications

1. Procédé pour la capture d'un échantillon de microorganismes ayant une surface hydrophobe, lesdits microorganismes étant des mycobactéries, lequel procédé consiste à mettre en contact les microorganismes avec un réactif de capture, lequel réactif de capture présente à la fois un caractère hydrophobe par lequel le réactif de capture se lie auxdites mycobactéries par interaction hydrophobe avec celles-ci et un caractère polaire et est du chlorure d'ammonium de poly-diallyldiméthyle (pDADMAC), ledit réactif de capture étant soit présent sur une surface et capturant lesdites mycobactéries sur celle-ci, soit étant présente en solution, ledit procédé consistant ensuite en outre à capturer lesdites mycobactéries sur une surface en liant ledit réactif de capture à ladite surface par interaction polaire entre ladite surface et ledit réactif de capture.

2. Procédé selon la revendication 1, dans lequel ledit réactif de capture comprend une longue chaîne hydrocarbonée portant de multiples sites polaires espacés le long de ladite chaîne.

3. Procédé selon la revendication 1 ou la revendication 2, dans lequel ledit réactif de capture est cationique.

4. Procédé selon la revendication 3, dans lequel ledit chlorure d'ammonium de poly-diallyldiméthyle (pDADMAC) a une masse moléculaire de 200 000 à 500 000.

5. Procédé selon la revendication 1, lequel procédé consiste à mettre en contact les mycobactéries avec un réactif de capture soluble qui comprend du poly-DADMAC, moyennant lequel le réactif de capture se lie auxdites mycobactéries et à capturer lesdites mycobactéries sur une surface en liant ledit réactif de capture à ladite surface.

6. Procédé selon l'une quelconque des revendications précédentes, dans lequel l'échantillon est mis en contact avec le réactif de capture en la présence d'un détergent qui renforce la sélectivité de la liaison des microorganismes souhaités.

7. Procédé selon la revendication 6, dans lequel le détergent comprend un amide d'acide aminé d'un acide gras.

8. Procédé selon la revendication 6, dans lequel le détergent comprend de la sarcosine de N-lauroyle.

9. Procédé selon l'une quelconque des revendications 6 à 8, dans lequel le détergent comprend un détergent Triton X.

10. Procédé pour la détection d'un microorganisme, consistant à capturer ledit microorganisme sur une surface grâce à un procédé selon l'une quelconque des revendications précédentes, laver ledit microorganisme capturé et détecter ledit microorganisme capturé sur ladite surface ou une fois éliminé de celle-ci.

11. Procédé selon la revendication 10, dans lequel la viabilité du microorganisme capturé est déterminée.

12. Procédé selon la revendication 11, dans lequel le microorganisme capturé est traité avec un médicament et la viabilité du microorganisme est déterminée pour établir si le médicament affecte la viabilité du microorganisme.

13. Kit d'analyse de mycobactéries comprenant soit (a) un réactif de capture soluble ayant à la fois un caractère hydrophobe par lequel le réactif de capture est capable de lier les mycobactéries devant être détectées par interaction hydrophobe avec celles-ci et un caractère polyionique, ledit réactif de capture étant du pDADMAC, un substrat ayant une surface pour capturer lesdites mycobactéries sur ladite surface en liant ledit réactif de capture à ladite surface par interaction polaire entre ladite surface et ledit réactif de capture, soit (b) un réactif de capture enduit sur une surface solide et immobilisé de ce fait sur celle-ci, ledit réactif de capture ayant à la fois un caractère hydrophobe et polyionique par lequel le réactif de capture est capable de lier les mycobactéries devant être détectées, ledit réactif de capture étant du pDADMAC,
et au moins l'un parmi:
- un phage capable d'infecter lesdites mycobactéries ;
- des amorces pour réaliser une amplification de l'acide nucléique génomique desdites mycobactéries ou dudit phage ;
- un colorant résistant à l'acide pour visualiser lesdites mycobactéries pour une inspection microscopique ; ou
- un anticorps pour lier lesdites mycobactéries ;
et contient éventuellement en outre :
- un milieu de culture pour cultiver lesdites mycobactéries,
- un réactif de détection pour utilisation dans la détection d'un métabolite produit lors de la culture desdites mycobactéries,
- un agent de détection spécifique pour les mycobactéries viables et/ou
- un ou plusieurs médicaments potentiellement aptes à affecter la viabilité desdites mycobactéries.

14. kit selon la revendication 13, dans lequel ledit réactif de capture est du pDADMAC ayant une masse moléculaire de 200 000 à 500 000.

15. Kit selon la revendication 13 ou la revendication 14, dans lequel ledit phage, lesdites amorces, ledit anticorps ou ledit agent de détection est (sont) spécifiques pour l'identification du complexe *M. tuberculosis, M. avium, M. intracellulare, M. paratuberculosis, M. leprae, M. kansasii, M. marinum* ou *M. fortuitum.*
